# EUROPEAN PATENT SPECIFICATION

(11) **EP 4 548 866 B1**
(45) Date of publication and mention of the grant of the patent: **11.02.2026**
(21) Application number: 24168924.9
(22) Date of filing: 08.04.2024
(51) Int. Cl.: A61B 17/80, A61B 17/82

(54) **AN INSERTED STERNUM PLATE**
EINGEFÜGTE STERNUMPLATTE
PLAQUE DE STERNUM INSÉRÉE

(30) Priority: 06.11.2023 CN 202311466895
(43) Date of publication of application: 07.05.2025
(73) Proprietor: Changzhou Waston Medical Appliance Co., Ltd, Changzhou, Jiangsu (CN)
(72) Inventor: Renmin, HU, Changzhou (CN)
(74) Representative: Musil, Dobroslav

(56) References cited:
- WO-A1-2017/102915
- WO-A1-2019/028969
- CN-U- 203 970 521
- US-A- 6 051 007

## Description

### Field of the invention

The present invention relates to the technical field of medical equipment, and in particular to an inserted sternum plate.

### State of the art

Midline thoracotomy refers to the use of a central sternotomy for cardiac and large vessel surgery and mediastinal tumor resection. Therefore, midline thoracotomy surgeons usually requires a complete or partial longitudinal incision of the sternum from the middle of it. After the surgery is completed, it is necessary to close and secure the sternum.

Currently, a sternal plate is usually implanted to close and secure the sternum after midline thoracotomy. The most basic structure of the sternal plate usually includes two connectors with claws. The claws on the two connectors are arranged oppositely. During use of the structure, after the sternum is closed through the two claws, firmware is used to connect the two connectors to achieve the closing and securing of the sternum.

WO2017102915A1 describes a sternal closure assembly for securing first and second lateral halves of a sternum. The assembly includes a first sliding body having a female portion and a second sliding body having a male portion, the male portion being telescopically mateable with the female portion. The male portion is capable of being fully introduced into the female portion only upon there being an elastic deformation of at least a part of at least one of the male and female portions, the male and female portions configured such that when the second body is mated with the first body the elastic deformation results in a forceful engagement between the male and female portions and also a forceful engagement between a first set of non-deforming teeth extending upward from an interconnecting bridge member of the first sliding body and a second set of non-deforming teeth extending upward from an interconnecting bridge member of the second sliding body. WO2017102915A1 relies on the snap-on elastic deformation bite action for primary fixation (see paragraph [0028]), lacking a secondary reinforcement scheme for the connection state, especially under high load or vibration conditions, there is a certain risk of loosening.

US6051007A describes a sternal closure device comprising first and second clamps. The first and second clamps have a generally tubular portion and the second clamp has a portion that is slidably receivable in the tubular portion, and a lock configured to retain said second clamp within said first clamp. A surgical instrument for laterally moving opposed sternal clamps toward one another is also disclosed. The instrument comprises first and second grasping members generally linearly slidably coupled to one another. US6051007A adopts a screw fixing structure (see Figure 13), which is an invasive mechanical fastening solution aimed at providing structural rigidity.

Existing sternal plates are connected through fasteners, and usually the connecting position of the fasteners cannot be changed. Therefore, the size of the sternal plate cannot be changed and each model of sternal plate can only be applied to the sternum within a certain size range. However, due to differences between individuals' size of sternum, the range of the use of such sternal plate is narrow and its versatility is poor.

### Principle of the invention

The technical problem to be solved by the present invention is: in order to solve the problem of poor versatility of the sternal plate in the current version, the present invention provides an inserted sternum plate, which uses an plug-in plate inserted in a hole to achieve the connection of the two connectors. The connecting length of the plug-in plate can be adjusted according to the patient's sternum size during use, so that the size of the plug-in sternum plate is made adjustable, expanding its scope of use and improving its versatility and solving the problem of poor versatility of the sternal plates in the current versions.

The technical solutions adopted by the present invention to solve the technical problems are:
an inserted sternum plate, which includes a primary connector and a secondary connector;
the said primary connector includes a plug-in plate;
the lower end surface of the plug-in plate is provided with a number of primary stopping teeth;
the secondary connector includes a connecting end;
the connecting end includes a connection end body, and a plug hole provided on the connecting end body;
the plug-in hole is provided with a bottom surface, which is situated, in the assembled state of the sternum plate, opposite the lower end surface of the plug-in plate with a number of primary stopping teeth;
secondary stopping teeth are provided on the bottom surface of the plug-in hole;
the plug-in plate is adapted to be engaged with the plug-in hole, so that the primary stopping teeth and the secondary stopping teeth can be engaged to achieve the connection between the primary connector and the secondary connector;
the number of the primary stopping teeth is greater than that of the secondary stopping teeth;
the inserted sternum plate is made of polyetheretherketone or carbon fiber reinforced polyetheretherketone,
limiting flanges are provided on both sides of the plug-in plate; **In** the said plug-in hole the first limiting groove adapted to the limiting flange are set,
the thickness of the limiting flange is smaller than the thickness of the first limiting groove, and a slot is formed between the limiting flange and the top surface of the first limiting slot; the connecting end also includes an insert adapted to the slot.

Optionally, the first outer surface of the primary stopping tooth is a bevel.

Optionally, the second outer surface of the secondary stopping tooth is a bevel.

Optionally, the front end of the plug-in plate is provided with a plug-in connector, which has an arc-shaped structure.

Optionally, the connecting section of the inserting piece has a tapered structure.

Optionally, the taper of the connecting section is measured in Morse taper.

Optionally, the said length of the connecting section is 7mm.

Optionally, hand-push parts are provided on both sides of the connecting end body.

The beneficial effects of the present invention are:
The inserted sternum plate provided by the present invention realizes the connection between the primary connector and the secondary connector by matching the plug-in plate with the plug-in hole. Since the number of the primary stopping teeth on the plug-in plate is more than that of the secondary stopping teeth in the hole, the connection length of the plug-in plate can be adjusted as per the size of the sternum, thereby making the inserted sternum plate applicable to sternums of various sizes, helping to expand its range of use and improving its versatility.

### Description of the drawings

The present invention will be further described below by use of the accompanying diagrams and exemplary cases.
Figure 1 is a schematic structural diagram of the inserted sternum plate in the present invention;
Figure 2 is an exploded view of the inserted sternum plate in the present invention;
Figure 3 is a schematic structural diagram of the primary connector in the present invention;
Figure 4 is schematic structural diagram of the primary connector in the present invention;
Figure 5 is a schematic structural diagram of the secondary connector in the present invention;
Figure 6 is a schematic structural diagram of the inserting piece in the present invention;
Figure 7 is a simplified assembly diagram of the primary connector and the plug-in hole in the present invention;
Figure 8 is a partial enlarged view of Area A in Figure 7.

In the figures: 1 is for primary connector; 11-plug-in plate; 111-primary stopping tooth; 1111-first outer surface; 112-plug connector; 113-plug-in flange; 12-first claw structure ; 2-secondary connector; 21-connecting end; 211-connecting end body; 2111-Hand-push part; 212-plug-in hole; 2121-first limiting groove; 213-secondary stopping tooth; 2131-second outer surface; 214-inserting piece; 215-slot; 22-second claw structure.

### Detailed description of the invention

The present invention will now be described in further detail. The embodiments described below are illustrative and are intended to explain the present invention and cannot be interpreted as a restriction to the present invention. Any other embodiments created based on this present invention by any ordinary technician in this industry without any creative work are all covered by the copyright of the present invention.

In the description of the present invention, it should be understood that the terms "primary (or first)" and "secondary (or second)" are only used to simplify the description, and shall not be interpreted to indicate or imply relative importance, or to implicitly indicate the number of technical features. Thus, features defined as "primary (or first)" and "secondary (or second)" may explicitly or implicitly include one or more of these features. In the description of the present invention, "plurality" means two or more than two, unless otherwise explicitly and specifically restricted.

In the present invention, unless otherwise expressly stipulated, if Feature 1 is "above" or "below" Feature 2, the two features may or may not be in direct contact. They can be in indirect contact with other feature or features in between. Furthermore, if Feature 1 is "above" or "on" Feature 2, they may or may not be in contact. Feature 1 can be directly above Feature 2 over the latter or is only positioned higher than the latter in altitude. If Feature 1 is "below", "under" or "underneath" Feature 2, former can be directly under the latter, or it is just lower in position than the latter in altitude.

In order to make the above objects, features and advantages of the present invention more obvious and understandable, specific embodiments of the present invention will be described in detail below with reference to the accompanying drawings.

The present invention is defined in claim 1 while preferred embodiments are set forth in the dependent claims.

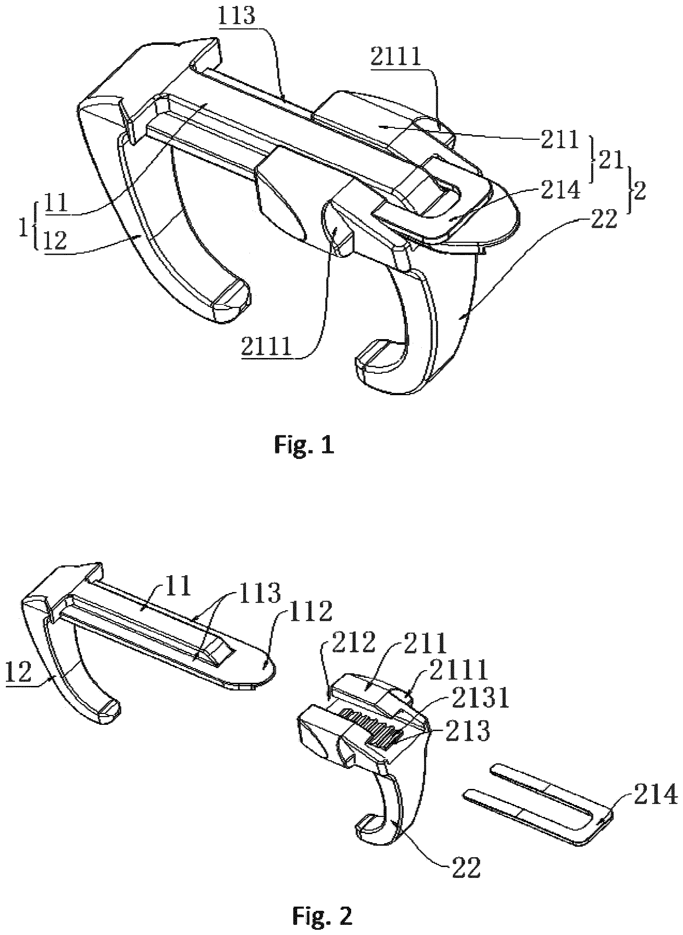

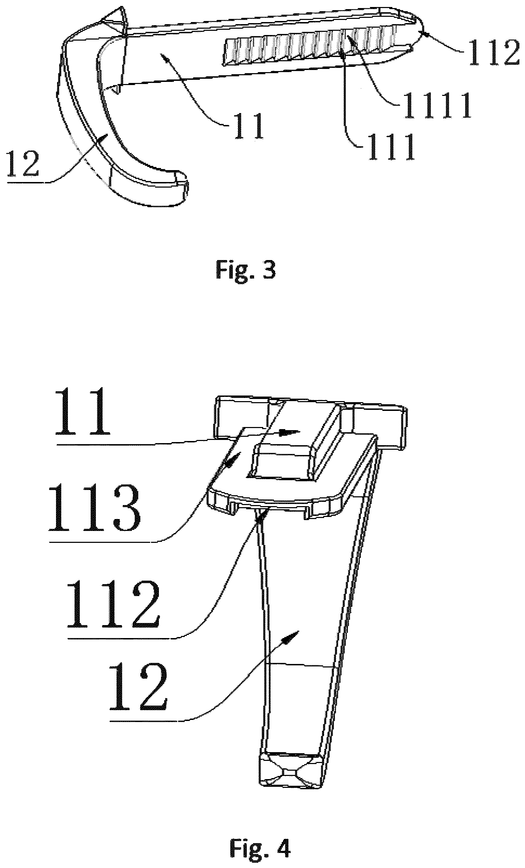

In order to solve the problem of poor versatility of the sternal plate in the current versions, the present invention provides an inserted sternum plate, as shown in Figures 1 to 8. The said sternum plate includes a primary connector 1 and a secondary connector 2, which are connected to close and secure the incised sternum. Specifically, the primary connector 1 includes a plug-in plate 11 through which it is connected to the secondary connector 2; preferably, the primary connector 1 also includes a first claw structure 12 connected to the plug-in plate 11; the lower end surface of the plug-in plate 11, that is, the end connected to the secondary connector 2 is provided with a plurality of primary stopping teeth 111; the secondary connector 2 includes a connecting end 21, through which it is connected to the primary connector 1; preferably the secondary connector 2 also includes a second claw structure 22 connected to the connecting end 21; the connecting end 21 includes a connecting end body 211, and a plug-in hole 212 provided on the connecting end body 211; the plug-in hole 212 is adapted to the plug-in plate 11; the bottom surface of the plug-in hole 212 is provided with secondary stopping teeth 213; the plug-in plate 11 is adapted to the plug-in hole 212, so as to realize the connection of the primary connector 1 and the secondary connector 2 through the engagement of the primary stopping teeth 111 and the secondary stopping teeth 213; specifically, during the assembly process, the plug-in plate 11 is inserted into the plug-in hole 212, and opposing thrusts are applied to the primary connector 1 and the secondary connector 2, so that the primary stopping teeth 111 and the secondary stopping teeth 213 are engaged, thereby connecting the primary connector 1 and the secondary connector 2.

After the primary connector 1 and the secondary connector 2 are installed at the sternum, when the patient coughs or exerts force on it, the connection between the primary connector 1 and the secondary connector 2 are pulled in the left and right direction, which pulls the primary connector 1 away from the secondary connector 2. Meanwhile, the secondary connector 2 tends to move in the direction away from the primary connector 1, resulting in slippage. To tackle this problem, the present invention preferably ensures that the directions of the arrangement of the primary teeth 111 and the secondary stopping teeth 213 are both perpendicular to the direction of the primary connector 1 and the secondary connector 2. With such a design, when the connection between the primary connector 1 and the secondary connector 2 is subject to a force in the left-right direction, both connectors will not move, thanks to the engagement of the primary and secondary stopping teeth 111 and 213. Thus, the slippage is avoided and the stability of the connection is improved.

In order to facilitate the closing and securing of the sternum, the present invention preferably requires that the first claw structure 12 and the second claw structure 22 are both hook-shaped structures suitable for closing and securing the sternum and; after the primary connector 1 and the secondary connector 2 are connected, the first claw structure 12 and the second claw structure 22 are arranged opposite each other on both sides of the sternum. A space suitable for accommodating the sternum is formed in between so as to realize closing and securing of the sternum.

Furthermore, in the present invention, it is preferred that the number of the primary stopping teeth 111 is greater than that of the secondary stopping teeth 213, so that the room accommodating the sternum can be adjusted by selecting the primary stopping teeth 111 in engagement with the secondary stopping teeth 213 on the plug-in plate 11 during the installation process. This allows each inserted sternum plate to be adapted to sternums of various sizes, which expands its scope of use and improving its versatility.

In the present invention, the preferred material of the inserted sternal plate is polyetheretherketone (PEEK), or carbon fiber reinforced with PEEK. Such a material is not only capable of ensuring the required mechanical properties of the sternal plate, but also can be cut with scissors off the residual materials after installation. On the other hand, using PEEK or PEEK modified materials, which allows for direct scissors operation, facilitates secondary thoracotomy in certain cases where tissues can grow on the initial implanted sternum plates.

The inserted sternum plate provided by the present invention realizes the connection between the primary connector and the secondary connector by matching the plug-in plate 11 with the plug-in hole 212. Since the number of the primary stopping teeth 111 on the plug-in plate 11 is more than that of the secondary stopping teeth 213 in the hole 212, the connection length of the plug-in plate can be adjusted as per the size of the sternum, thereby making the inserted sternum plate applicable to sternums of various sizes, helping to expand its range of uses and improving its versatility.

In order to facilitate the connection between the plug-in plate 11 and the connecting end 21, the present invention prefers that the outer side of the primary stopping tooth 111, denoted as the first outer side 1111, is a bevel; specifically, in this article, the outer side of the primary stopping tooth 111 refers to the side of the primary stopping teeth 111 away from the first holding claw structure 12, after assembling the plug-in plate 11 and the connecting end 21; The structure with the outer outer side 1111 being set as a bevel helps reduce the resistance during the assembly process, thereby reducing the difficulty of assembly.

Similarly, the present invention prefers that the outer side of the secondary stopping teeth 213, denoted as the first outer side 2131, is a bevel; specifically, in this article, the outer side of the secondary stopping tooth 213 refers to the side of the primary stopping teeth 213 away from the second holding claw structure 22, after assembling the plug-in plate 11 and the connecting end 21; The structure with the outer outer side 2131 being set as a bevel helps reduce the resistance during the assembly process, thereby reducing the difficulty of assembly.

To facilitate the assembly, the present invention prefers that the front end of the plug-in plate 11, that is, the end of the plug-in plate 11 away from the first claw structure 12, is provided with a plugging connector 112, which can be triangular, arc-shaped, circular, etc. In order to take into account both the convenience of assembly and the aesthetics, the plug connector 112 of the present invention preferably has an arc-shaped structure.

It should be noted that after the primary connector 1 and the primary connector 2 are assembled, the excess portion of one end of the plug connector 112 on the plugging plate 11 can be cut, and the specific cutting length is determined according to the size of the sternum.

Furthermore, in the present invention, limiting flanges 113 are provided on both sides of the plug-in plate 11; a limiting groove matching the limiting flange 113 is provided in the plug-in hole 212, which is marked as the first limiting groove 2121; In the present invention, it is preferred that the number of the limiting flange 113 and the first limiting groove 2121 is two; during the assembly process, by inserting the limiting flange 113 into the first limiting groove 2121 and then pushing them, this helps improve assembly accuracy, making the assembly ease and efficient.

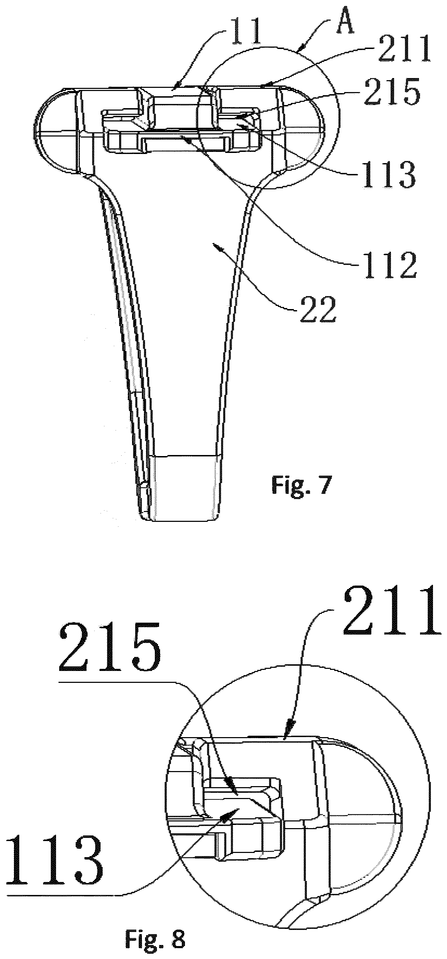

During the assembly process of the inserted sternum plate of the present invention, the plug-in plate 11 needs to be inserted into the plug-in hole 212. Due to the existence of the primary stopping teeth 111 and the secondary stopping teeth 213, it is necessary to exert a great the thrust during the assembly, making it difficult to assemble. To solve this problem, the present invention makes the thickness of the limiting flange 113 smaller than that of the first limiting groove 2121, that is, the gap between the top of the first limiting groove 2121 and its bottom is greater than the thickness of the flange 113, so that the limiting flange 113 can be easily inserted into the first limiting groove 2121 during the assembly process, and then simply engage the primary stopping teeth 111 with the secondary stopping teeth 213, which greatly reduces the assembly difficulty. Since the thickness of the limiting flange 113 is smaller than that of the first limiting groove 2121, after the primary connector 1 and secondary connector 2 are assembled through the engagement of primary stopping teeth 111 and secondary stopping teeth 213, there is a clearance between the limiting flange 113 and the top of the first limiting groove 2121, which form a slot 215, as shown in Figures 7 and 8. In order to ensure the stability of the sternal plate structure after the assembly is completed, the present invention prefers the connecting end 21 also includes an insert 214 that is adapted to the slot 215. By inserting the insert 214 into the slot 215 to fill the gap, the risk of the plug-in plate 11 falling off the plug hole 212 is reduced, and the safety use is further improved. During use, after inserting the insert piece 214 into the slot 215, the portion of the insert piece 214 located outside the slot 215 can be cut, if required, and the cutting length can be determined by the actual needs.

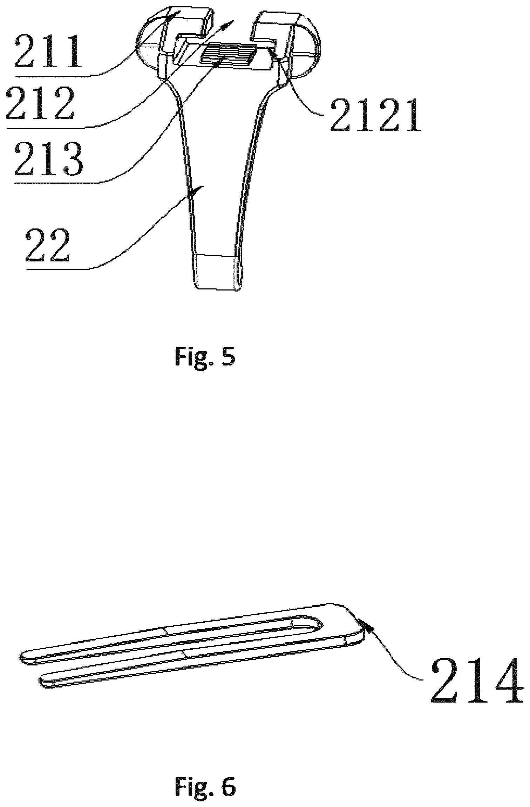

In order to facilitate assembly, as shown in Figure 6, the present invention preferably incorporates the connecting section of the insert piece 214, that is, the section of the insert piece 214 suitable for being inserted into the slot 215 has a tapered structure. Correspondingly, the inner cavity of the slot 215 is also tapered structure.

The connecting section is designed to be a tapered structure. Taper fitting is adopted between the insert piece 214 and the slot 215. This reduces the difficulty in disassembling the insert piece 214 from the slot 215, making it easier to disassemble the insert sternum plate.

In order to further improve the stability of the connection, the present invention prefers that the taper of the connecting section is a Morse taper, so that the static fit between the insert piece 214 and the slot 215 can be achieved through the Morse taper and the principle of friction, thereby achieving the self-locking of the insert 214, ensuring the stability of the installation without the need for additional components.

Furthermore, in the present invention, the preferred length of the connecting section is 7 mm.

In order to facilitate the operation, the present invention preferably is provided with hand-push part 2111 on both sides of the connection end body 211. The hand-push part 2111 can be set in any structural form suitable for the operator to hold and push hard when needed, so as to facilitate the operator's assembly. During the process, the hand-push part 2111 is used to exert a thrust toward the primary connector 1 on the secondary connector 2, thereby reducing the difficulty in operation.

Taking the above-mentioned ideal embodiments of the present invention as reference and through the above description, relevant workers can make various changes and modifications without deviating from the scope of the technical idea of the present invention. The technical scope of the present invention is not limited to the content in the description and must be determined based on the scope of the claims.

## Claims

1. An inserted sternum plate, includes
a primary connector (1) and a secondary connector (2);
the primary connector (1) includes a plug-in plate (11);
the lower end surface of the plug-in plate (11) is provided with a number of primary stopping teeth (111);
the secondary connector (2) includes a connecting end (21);
the connecting end (21) includes a connection end body (211), and a plug hole (212) provided on the connection end body (211);
the plug-in hole (212) is provided with a bottom surface, which is situated, in the assembled state of the sternum plate, opposite the lower end surface of the plug-in plate (11) with a number of primary stopping teeth (111);
secondary stopping teeth (213) are provided on the bottom surface of the plug-in hole (212);
the plug-in plate (11) is adapted to be engaged with the plug-in hole (212), so that the primary stopping teeth (111) and the secondary stopping teeth (213) can be engaged to achieve the connection between the primary connector (1) and the secondary connector (2);
the number of the primary stopping teeth (111) is greater than that of the secondary stopping teeth (213);
the inserted sternum plate is made of polyetheretherketone or carbon fiber reinforced polyetheretherketone,
limiting flanges (113) are provided on both sides of the plug-in plate (11); In the said plug-in hole (212) the first limiting groove (2121) adapted to the limiting flange (113) are set,
the thickness of the limiting flange (113) is smaller than the thickness of the first limiting groove (2121), and a slot (215) is formed between the limiting flange (113) and the top surface of the first limiting slot (2121); the connecting end (21) also includes an insert (214) adapted to the slot (215).

2. The inserted sternum plate, as described in claim 1, is **characterized in that** the first outer surface (1111) of the primary stopping teeth (111) is a bevel.

3. The inserted sternum plate, as described in claim 1, is **characterized in that** the first outer surface (2131) of the secondary stopping teeth (213) is a bevel.

4. The inserted sternum plate, as described in claim 1, is **characterized in that** the front end of the plug-in plate (11) is provided with a plug connector (112), which has an arc-shaped structure.

5. The inserted sternum plate as described in claim 1, is **characterized in that** the connecting section of the insert (214) has a tapered structure.

6. The inserted sternum plate, as described in claim 5, is **characterized by** the fact that the taper of its connecting section is measured by Morse taper.

7. The inserted sternum plate, as described in claim 5, is **characterized by** the fact that the length of its connecting section is 7mm.

8. The inserted sternum plate, as described in any one of the claims 1 to 7, is **characterized in that** hand-push insert (214) are provided on both sides of the connecting end's body (2111).

## Patentansprüche

1. Eingelegte Sternumplatte, die einen primären Steckverbinder (1) und einen sekundären Steckverbinder (2) aufweist;
der primäre Steckverbinder (1) weist eine Steckplatte (11) auf;
die untere Endoberfläche der Steckplatte (11) weist eine Reihe von primären Anschlagzähnen (111) auf;
der sekundäre Steckverbinder (2) weist ein Verbindungsende (21) auf;
das Verbindungsende (21) weist einen Verbindungsendkörper (211) und eine auf dem Verbindungsendkörper (211) ausgebildete Stecköffnung (212) auf;
die Stecköffnung (212) weist einen Boden auf, der sich im zusammengebauten Zustand der Sternumplatte gegen die untere Endoberfläche der Steckplatte (11) mit einer Reihe von primären Anschlagzähnen (111) befindet;
am Boden der Stecköffnung (212) sind die sekundären Anschlagzähne (213) angeordnet;
die Steckplatte (11) ist zum Hineinschieben in die Stecköffnung (212) angepasst, sodass die primären Anschlagzähne (111) und die sekundären Anschlagzähne (213) zusammenwirken können, um eine Verbindung zwischen dem primären Steckverbinder (1) und dem sekundären Steckverbinder (2) herzustellen;
die Anzahl der primären Anschlagzähne (111) ist größer als die Anzahl der sekundären Anschlagzähne (213);
eingelegte Sternumplatte ist aus Polyetheretherketon oder kohlenstofffaserverstärktem Polyetheretherketon hergestellt;
an beiden Seiten der Steckplatte (11) sind Begrenzungsflansche (113) angeordnet; in der genannten Stecköffnung (212) sind erste Begrenzungsnuten (2121) angeordnet, die an den Begrenzungsflansch (113) angepasst sind,
die Dicke des Begrenzungsflansches (113) ist geringer als die Dicke der ersten Begrenzungsnut (2121) und zwischen dem Begrenzungsflansch (113) und der Oberseite der ersten Begrenzungsnut (2121) wird ein Spalt (215) ausgebildet; das Verbindungsende (21) weist außerdem einen Einsatz (214) auf, der an den Spalt (215) angepasst ist.

2. Eingelegte Sternumplatte nach Anspruch 1, **dadurch gekennzeichnet, dass** die erste Außenoberfläche (1111) der primären Anschlagzähne (111) abgeschrägt ist.

3. Eingelegte Sternumplatte nach Anspruch 1, **dadurch gekennzeichnet, dass** die erste Außenoberfläche (2131) der sekundären Anschlagzähne (213) abgeschrägt ist.

4. Eingelegte Sternumplatte nach Anspruch 1, **dadurch gekennzeichnet, dass** das vordere Ende der Steckplatte (11) einen Steckverbinder (112) aufweist, der eine Bogenstruktur aufweist.

5. Eingelegte Sternumplatte nach Anspruch 1, **dadurch gekennzeichnet, dass** der Verbindungsabschnitt des Einsatzes (214) eine sich verjüngende Struktur aufweist.

6. Eingelegte Sternumplatte nach Anspruch 5, **dadurch gekennzeichnet, dass** die Verjüngung ihres Verbindungsabschnitts mittels eines Morsekonus gemessen wird.

7. Eingelegte Sternumplatte nach Anspruch 5, **dadurch gekennzeichnet, dass** die Länge ihres Verbindungsabschnitts 7 mm beträgt.

8. Eingelegte Sternumplatte nach einem der Ansprüche 1 bis 7, **dadurch gekennzeichnet, dass** an beiden Seiten des Verbindungsendes des Körpers (2111) manuell einzusteckende Einsätze (214) angeordnet sind.

## Revendications

1. Plaque sternale insérée comprenant un connecteur primaire (1) et un connecteur secondaire (2) ;
le connecteur primaire (1) comprend une plaque d'insertion (11) ;
la surface d'extrémité inférieure de la plaque d'insertion (11) est pourvue d'une série de dents d'arrêt primaires (111) ;
le connecteur secondaire (2) comprend une extrémité de connexion (21) ;
l'extrémité de connexion (21) comprend un corps d'extrémité de connexion (211) et une ouverture d'insertion (212) formée sur le corps d'extrémité de connexion (211) ;
l'ouverture d'insertion (212) est pourvue d'un fond qui, à l'état assemblé de la plaque sternale, se trouve en face de la surface d'extrémité inférieure de la plaque d'insertion (11) avec une série de dents d'arrêt primaires (111) ;
des dents d'arrêt secondaires (213) sont disposées sur le fond de l'ouverture d'insertion (212) ;
la plaque d'insertion (11) est adaptée pour être insérée dans l'ouverture d'insertion (212) de telle sorte que les dents d'arrêt primaires (111) et les dents d'arrêt secondaires (213) puissent coopérer pour réaliser la connexion entre le connecteur primaire (1) et le connecteur secondaire (2) ;
le nombre de dents d'arrêt primaires (111) est supérieur au nombre de dents d'arrêt secondaires (213) ;
la plaque sternale insérée est fabriquée en polyétheréthercétone ou en polyétheréthercétone renforcée de fibres de carbone,
des brides de limitation (113) sont disposées des deux côtés de la plaque d'insertion (11) ; des premières rainures de limitation (2121) adaptées à la bride de limitation (113) sont disposées dans ladite ouverture d'insertion (212) ;
l'épaisseur de la bride de limitation (113) est inférieure à l'épaisseur de la première rainure de limitation (2121) et une fente (215) est formée entre la bride de limitation (113) et la surface supérieure de la première rainure de limitation (2121) ; l'extrémité de connexion (21) comprend également un insert (214) adapté à la fente (215).

2. Plaque sternale insérée telle que décrite dans la revendication 1, **caractérisée en ce que** la première surface extérieure (1111) des dents d'arrêt primaires (111) est biseautée.

3. Plaque sternale insérée, telle que décrite dans la revendication 1, **caractérisée en ce que** la première surface extérieure (2131) des dents d'arrêt secondaires (213) est biseautée.

4. Plaque sternale insérée, telle que décrite dans la revendication 1, **caractérisée en ce que** l'extrémité avant de la plaque d'insertion (11) est pourvue d'un connecteur d'insertion (112) qui présente une structure arquée.

5. Plaque sternale insérée, telle que décrite dans la revendication 1, **caractérisée en ce que** la partie de connexion de l'insert (214) présente une structure effilée.

6. Plaque sternale insérée, telle que décrite dans la revendication 5, **caractérisée en ce que** le rétrécissement de sa partie de connexion est mesuré à l'aide d'un cône Morse.

7. Plaque sternale insérée, telle que décrite dans la revendication 5, **caractérisée en ce que** la longueur de sa partie de connexion est de 7mm.

8. Plaque sternale insérée, telle que décrite dans l'une des revendications de 1 à 7, **caractérisée en ce que** des inserts inséré manuellement (214) sont disposés des deux côtés de l'extrémité de connexion du corps (2111).
